# EUROPÄISCHE PATENTSCHRIFT

(11) **EP 0 576 997 B1**
(45) Veröffentlichungstag und Bekanntmachung des Hinweises auf die Patenterteilung: **18.09.1996**
(21) Anmeldenummer: 93110057.2
(22) Anmeldetag: 24.06.1993
(51) Int. Cl.: C09B 50/00

(54) **Verfahren zur Herstellung von Aminoformazanen**
Method of preparing aminoformazanes
Procédé de préparation d'aminoformazanes

(30) Priorität: 01.07.1992 DE 4221543
(43) Veröffentlichungstag der Anmeldung: 05.01.1994
(73) Patentinhaber: BASF Aktiengesellschaft, 67063 Ludwigshafen (DE)
(72) Erfinder: Wiesenfeldt, Matthias, Dr., D-6704 Mutterstadt (DE); Pandl, Klaus, Dr. c/o BASF Suemebank, TR-41810 Gebze-Kocaeli (TR); Patsch, Manfred, Dr., D-6706 Wachenheim (DE)

(56) Entgegenhaltungen:
- EP-A- 0 280 139
- EP-A- 0 402 318

## Beschreibung

Die vorliegende Erfindung betrifft ein neues Verfahren zur Herstellung von Metall-Aminoformazanen durch alkalische Hydrolyse der entsprechenden acylierten Aminoderivaten.

Nach einer aus der EP-A-280 139 bekannten Methode werden aminogruppenhaltige Formazanfarbstoffe durch Umsetzung von einem Arylhydrazon mit einem diazotierten o-Hydroxyaminobenzol hergestellt. Um eine selektive Reaktion zu gewährleisten, müssen die im Endprodukt vorhandenen Aminogruppen jedoch vorher mit einer Schutzgruppe versehen werden. Hierfür wird zumeist eine Acylgruppe verwendet. Nach der Bildung des Formazanfarbstoffes muß diese Schutzgruppe durch Verseifung wieder abgespalten werden. Aufgrund der langsamen Abspaltung der Acylfunktion im alkalischen Medium sind hierbei lange Reaktionszeiten notwendig, was insbesondere zu Ausbeuteverlusten durch Bildung von Nebenprodukten führt.

Bei der nachfolgenden Umsetzung mit hydrolyseempfindlichen faserreaktiven Verbindungen, z.B. mit Chlortriazinen, erfolgt bei Verunreinigung mit Nebenprodukten verstärkt Hydrolyse des Reaktivrestes. Zur Vermeidung von Ausbeuteverlusten sind daher oftmals aufwendige Reinigungs- und Trennverfahren notwendig.

In der EP-A-402 318 wird ein Verfahren zur Vermeidung von Nebenprodukten bei der Hydrolyse beschrieben, indem die Aminofunktion in eine Urethangruppe übergeführt wird. Diese Methode ist jedoch aufgrund der relativ leichten Abspaltbarkeit der Schutzgruppe während der Kupplungsreaktion limitiert.

Aufgabe der vorliegenden Erfindung war es nun, ein neues Verfahren zur Herstellung von Aminoformazanen bereitzustellen, daß von acylierten Aminoformazanen ausgeht und bei dem die Abspaltung der Acylschutzgruppe schnell und auf einfache Weise durchzuführen ist.

Es wurde gefunden, daß die Herstellung von Aminoformazanen der Formel I 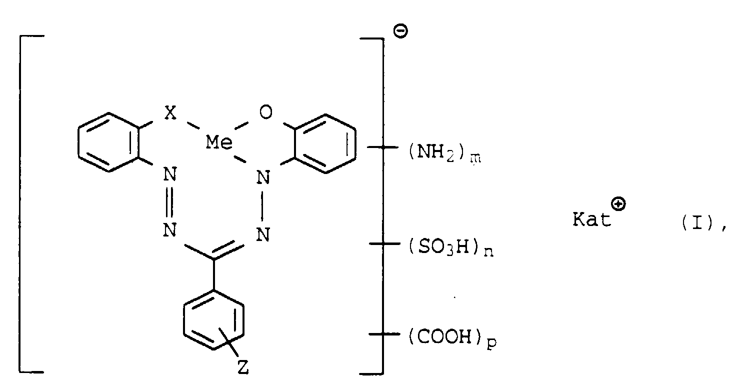 in der
- X: Sauerstoff oder einen Rest der Formel CO-O oder SO₂-O,
- Me: Kupfer oder Nickel,
- Z: Wasserstoff, Halogen oder Hydroxy,
- Kat^{⊕}: das Äquivalent eines Kations,
- m: 1 oder 2,
- n: 1, 2, 3 oder 4 und
- p: 0, 1 oder 2 bedeuten, mit der Maßgabe, daß die Summe von n+p 1 bis 4 beträgt,
durch alkalische Hydrolyse von acylierten Aminoformazanen der Formel II 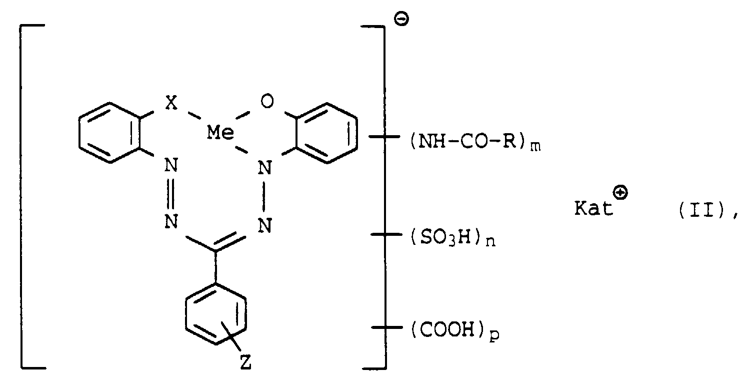 in der R Wasserstoff oder C₁-C₄-Alkyl bedeutet und X, Me, Z, Kat^{⊕} , m, n und p jeweils die obengenannte Bedeutung besitzen, in wäßrigem Medium vorteilhaft gelingt, wenn man die Hydrolyse bei einer Temperatur von 120 bis 170°C und einem Druck von 3 bis 5 bar in Gegenwart von 1,5 bis 3 mol eines Alkalihydroxids, bezogen auf 1 mol acyliertes Aminoformazan der Formel II, durchführt.

Die Herstellung von formylierten Aminoformazanen ist in der älteren europäischen Patentanmeldung EP-A-574 799 beschrieben.

Kat^{⊕} ist das Äquivalent eines Kations und leitet sich von einem Proton oder von Metall- oder Ammoniumionen ab. Metallionen sind insbesondere die Lithium-, Natrium- oder Kaliumionen. Ammoniumionen sind entweder unsubstituierte oder substituierte Ammoniumkationen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch Hydroxylgruppen substituiert und/oder durch Sauerstoffatome in Etherfunktion unterbrochen sein kann.

Insbesondere sind Lithium-, Natrium- oder Kaliumionen zu nennen, wobei Natriumionen besonders hervorzuheben sind.

Reste Z in Formel I sind z.B. Fluor, Chlor oder Brom.

Geeignete Alkalihydroxide sind z.B. Lithium-, Natrium- oder Kaliumhydroxid, wobei die Anwendung von Natrium- oder Kaliumhydroxid und insbesondere von Natriumhydroxid hervorzuheben ist.

Bevorzugt ist eine Verfahrensweise bei der die Hydrolyse bei einer Temperatur von 140 bis 160°C vorgenommen wird.

Bevorzugt ist weiterhin eine Verfahrensweise, bei der die Hydrolyse bei einem Druck von 3 bis 4 bar vorgenommen wird.

Bevorzugt ist weiterhin eine Verfahrensweise, bei der man die Hydrolyse in Gegenwart von 2 bis 3 mol Alkalihydroxid, bezogen auf 1 mol acyliertes Aminoformazan der Formel II durchführt.

Bevorzugt ist weiterhin ein Verfahren zur Herstellung von Aminoformazanen der Formel I, in der X den Rest der Formel CO-O und Me Kupfer bedeuten.

Bevorzugt ist weiterhin ein Verfahren, bei der man acyliertes Aminoformazan der Formel II hydrolysiert, in der R Wasserstoff oder Methyl, dabei insbesondere Methyl bedeutet.

Bevorzugt ist weiterhin eine Verfahrensweise zur Herstellung von Aminoformazanen der Formel I, in der Z Wasserstoff bedeutet.

Bevorzugt ist weiterhin eine Verfahrensweise zur Herstellung von Aminoformazanen der Formel I, in der p 0 bedeutet.

Besonders zu nennen ist eine Verfahrensweise, bei der man ein acyliertes Aminoformazan der Formel IIa 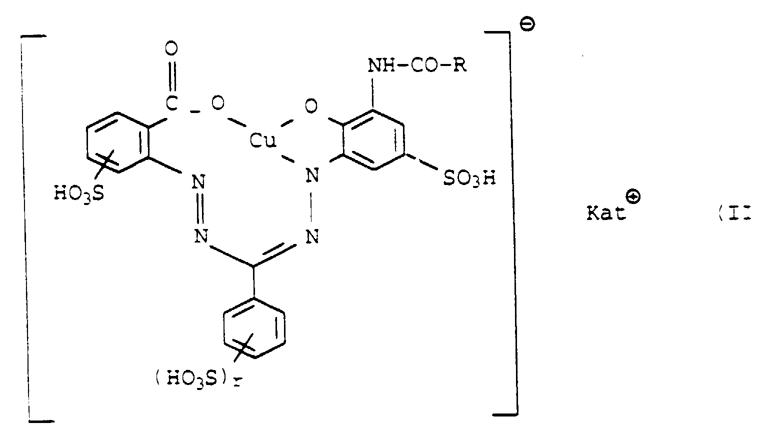 in der r 0, 1 oder 2 bedeutet und Kat^{⊕} und R jeweils die obengenannte Bedeutung besitzen, hydrolysiert.

Das erfindungsgemäße Verfahren wird in wäßrigem Medium vorgenommen. Dabei kann man z.B. acylierte Aminoformazane der Formel II anwenden, die nach ihrer Synthese zwischenisoliert worden sind. Vorteilhafter ist es jedoch, direkt das bei der Herstellung der acylierten Aminoformazane der Formel II anfallende wäßrige Reaktionsgemisch für die erfindungsgemäße Hydrolyse zu verwenden.

Je Gewichtsteil acyliertes Aminoformazan II sind dabei in der Regel 3 bis 10 Gewichtsteile, vorzugsweise 4,5 bis 6,5 Gewichtsteile, Wasser anwesend.

Das neue Verfahren wird zweckmäßig so durchgeführt, daß man eine wäßrige Suspension des acylierten Aminoformazans zusammen mit dem Alkalihydroxid, das üblicherweise in Form von 20 bis 50 gew.-%iger wäßriger Lauge zur Anwendung kommt, in eine geeignete Druckapparatur, z.B. in einen Autoklaven, gibt und für 1 bis 7 Stunden auf eine Temperatur von 120 bis 170°C erhitzt, wobei sich ein Druck von 3 bis 5 bar einstellt. Nach beendeter Umsetzung wird auf eine Temperatur von 20 bis 70°C abgekühlt und die Druckapparatur entspannt. Das Reaktionsgemisch wird dann klärfiltriert und kann direkt für weitere Umsetzungen, z.B. für die Umsetzung mit reaktiven Ankern, wie Cyanurchlorid, zu Reaktivfarbstoffen verwendet werden.

Das Zielprodukt kann natürlich auch isoliert werden, beispielsweise in dem man es aussalzt, abtrennt und trocknet.

Für die Umsetzung mit dem Reaktivanker wird das Reaktionsgemisch in der Regel auf einen pH-Wert von 4,5 bis 5,0 gestellt und anschließend auf an sich bekanntem Wege mit der Ankerkomponente zum Reaktivfarbstoff umgesetzt.

Mittels des neuen Verfahrens, das sowohl in kontinuierlicher als auch in diskontinuierlicher Arbeitsweise vorgenommen werden kann, werden die Aminoformazane der Formel I selektiv in hoher Ausbeute und Reinheit erhalten. Diese Produkte sind, wie oben ausgeführt, wertolle Zwischenprodukte für die Synthese von Formazan-Reaktivfarbstoffen und können ohne aufwendige Reinigung oder Abtrennung von Nebenprodukten mit deutlich erhöhter Raum-Zeit-Ausbeute in die entsprechenden Reaktivfarbstoffe übergeführt werden.

Die folgenden Beispiele sollen die Erfindung näher erläutern.

### Beispiel 1

a) 13 g (0,05 mol) 3-Acetylamino-4-hydroxybenzolsulfonsäure (Val 260) wurden in 100 ml Wasser durch Zugabe von 3 ml 50 gew.-%iger wäßriger Natronlauge gelöst. Nach Zugabe von 12,5 ml Salzsäure (M 100) wurden bei 0 bis 5°C 15 ml (0,05 mol) wäßrige Natriumnitritlösung (M 300) zugetropft. Das Reaktionsgemisch wurde 2 Stunden bei 0 bis 5°C gerührt. Das überschüssige Nitrit wurde durch Zugabe von Amidosulfonsäure entfernt. Die Reaktionslösung wurde anschließend bei 15 bis 20°C zu einer Lösung von 33 g (0,05 mol) Hydrazon der Formel 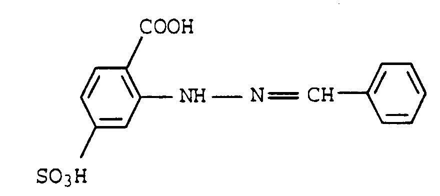 (Val 660) in 50 ml Wasser und 11 ml 50 gew.-%iger wäßriger Natronlauge und 12,5 g Kupfersulfat getropft. Die erhaltene Suspension, die das acetylierte Aminoformazan der Formel 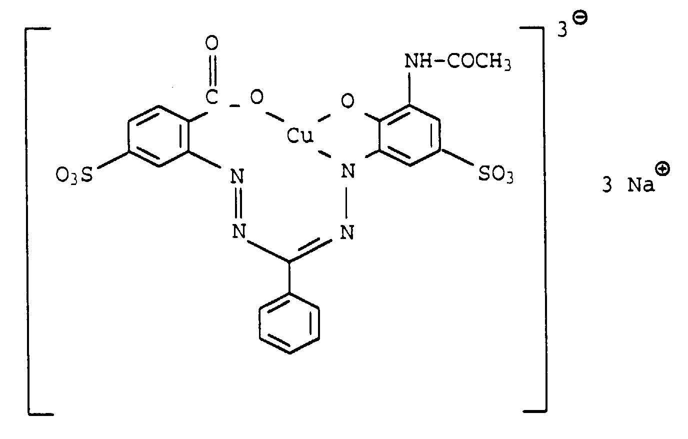 enthielt, wurde in den folgenden Beispielen ohne weitere Reinigung weiter verwendet.
b) Zu der unter Beispiel 1a) beschriebenen Suspension wurden 8,3 ml (0,15 mol) 50 gew.-%ige wäßrige Natronlauge gegeben. Die resultierende Mischung wurde in einem 1 l-Autoklaven 3 Stunden auf 140°C erhitzt (Druck ca. 4 bar). Nach Beendigung der Reaktion wurde auf 70°C abgekühlt, entspannt und die Reaktionslösung klärfiltriert. Man erhielt 747,4 g einer wäßrigen Lösung, die die freie Aminoverbindung der Formel 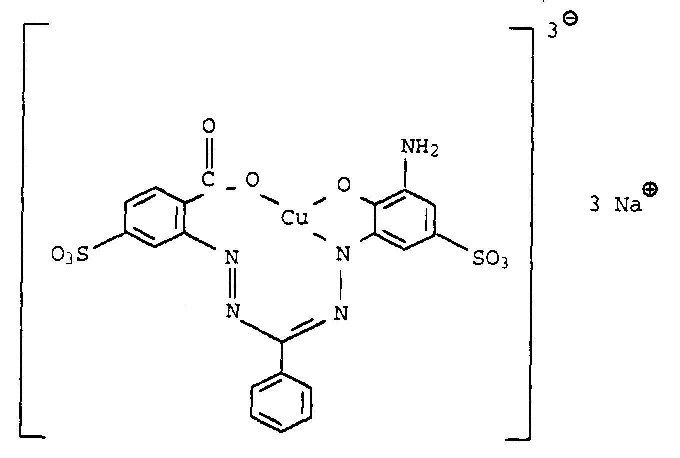 enthielt.
c) Die unter Beispiel 1b) erhaltene Lösung wurde mit Salzsäure auf einen pH-Wert von 5 gestellt. Hierzu wurde bei 5°C eine Lösung von 7,4 g (0,04 mol) Cyanurchlorid in 85 ml Aceton gegeben. Anschließend wurde auf 20°C erwärmt und durch Aussalzen mit 25 g Kaliumchlorid und 75 g Natriumchlorid das Produkt ausgefällt. Man erhielt 39,4 g der Verbindung der Formel 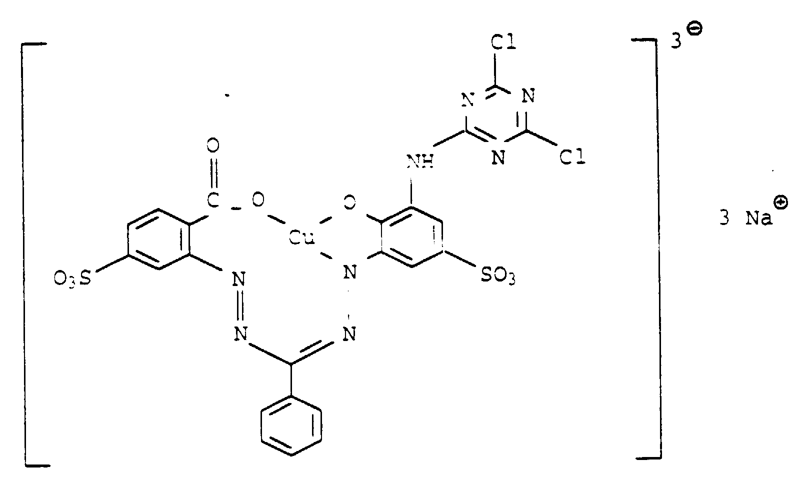 mit einem Gehalt von 78 %. Die Ausbeute betrug 76 % d. Th.

### Beispiel 2

Zu der nach Beispiel 1a) hergestellten Suspension wurden 8,3 ml (0,15 mol) 50 gew.-%ige wäßrige Natronlauge gegeben. Die resultierende Mischung wurde in einem 1 l Autoklaven 2 Stunden auf 150°C erhitzt (Druck: ca. 4 bar). Nach Beendigung der Reaktion wurde auf 70°C abgekühlt, entspannt und die Reaktionslösung klärfiltriert. Man erhielt 812,6 g Farbstofflösung. Diese wurde mit Salzsäure auf einen pH-Wert von 5 eingestellt. Hierzu wurde bei 5°C eine Lösung von 7,4 g (0,04 mol) Cyanurchlorid in 85 ml Aceton gegeben. Anschließend wurde auf 20°C erwärmt und durch Aussalzen mit 25 g Kaliumchlorid und 75 g Natriumchlorid das Produkt ausgefällt. Nach Absaugen und Trocknen erhielt man 42,37 g des in Beispiel 1c) beschriebenen Farbstoffs mit einem Gehalt von 79 %. Die Ausbeute betrug 83 % d. Th.

### Beispiel 3

a) 52 g (0,2 mol) 3-Acetylamino-4-hydroxybenzolsulfonsäure (Val 260) wurden in 400 ml Wasser durch Zugabe von 12 ml 50 gew.-%iger wäßriger Natronlauge gelost. Nach Zugabe von 50 ml Salzsäure (M 100) wurden bei 0 bis 5°C 60 ml (0,2 mol) wäßrige Natriumnitritlösung (M 300) zugetropft. Das Reaktionsgemisch wurde 2 Stunden bei 0 bis 5°C gerührt. Das überschüssige Nitrit wurde durch Zugabe von Amidosulfosäure entfernt. Die Reaktionslösung wurde anschließend bei 15 bis 20°C zu einer Lösung von 132 g (0,2 mol) des unter Beispiel la) genannten Hydrazons (Val 660) in 200 ml Wasser und 39 ml 50 gew.-%iger wäßriger Natronlauge und 50 g Kupfersulfat getropft. Die erhaltene Suspension, die das unter Beispiel la) angegebene Aminoformazan enthielt, wurde in den folgenden Beispielen ohne weitere Reinigung weiterverwendet.
b) Zu der unter Beispiel 3a) hergestellten Suspension wurden 33 ml (0,6 mol) 50 gew.-%ige wäßrige Natronlauge gegeben. Die resultierende Mischung wurde in einem 2 1-Autoklaven eine Stunde auf 160°C erhitzt (Druck: ca. 4 bar). Nach Beendigung der Reaktion wurde auf 70°C abgekühlt, entspannt und die Reaktionslösung klärfiltriert. Man erhielt 1853 g Farbstofflösung, die die unter Beispiel 1b) angegebene freie Aminoverbindung enthielt.
c) Die unter Beispiel 3c) erhaltene Lösung wurde mit Salzsäure auf einen pH-Wert von 5 eingestellt. Hierzu wurde bei 5°C eine Lösung von 35 g (0,19 mol) Cyanurchlorid in 90 ml Aceton gegeben. Anschließend wurde auf 20°C erwärmt und durch Aussalzen mit 100 g Kaliumchlorid und 300 g Natriumchlorid das Produkt ausgefällt. Nach Absaugen und Trocknen erhielt man 157,6 g des unter Beispiel lc) beschriebenen Reaktivfarbstoffs mit einem Gehalt von 67,6 %. Die Ausbeute betrug 66 % d. Th.

### Beispiel 4

Zu der unter Beispiel 3a) hergestellten Suspension wurden 33 ml (0,6 mol) 50 gew.-%ige wäßrige Natronlauge gegeben. Die resultierende Mischung wurde in einem 2 1-Autoklaven unter Stickstoff-Atmosphäre eine Stunde auf 160°C erhitzt (Druck: ca. 4 bar). Nach Beendigung der Reaktion wurde auf 70°C abgekühlt, entspannt und die Reaktionslösung klärfiltriert. Man erhielt 1880 g Farbstofflösung. Diese wurde mit Salzsäure auf einen pH-Wert von 5 eingestellt. Hierzu wurde bei 5°C eine Lösung von 35 g (0,19 mol) Cyanurchlorid in 90 ml Aceton gegeben. Anschließend wurde auf 20°C erwärmt und durch Aussalzen mit 100 g Kaliumchlorid und 300 g Natriumchlorid das Produkt ausgefällt. Nach Absaugen und Trocknen erhielt man 178,5 g des unter Beispiel 1c) beschriebenen Reaktivfarbstoffs mit einem Gehalt von 75,4 %. Die Ausbeute betrug 83 % d. Th.

### Beispiel 5

a) 52 g (0,2 mol) 3-Acetylamino-4-hydroxybenzolsulfonsäure (Val 260) wurden in 400 ml Wasser durch Zugabe von 12 ml 50 gew.-%iger wäßriger Natronlauge gelöst. Nach Zugabe von 50 ml Salzsäure (M 100) wurden bei 0 bis 5°C 60 ml (0,2 mol) wäßrige Natriumnitritlösung (M 300), zugetropft. Das Reaktionsgemisch wurde 2 Stunden bei 0 bis 5°C gerührt. Das überschüssige Nitrit wurde durch Zugabe von Amidosulfonsäure entfernt. Die Reaktionslösung wurde anschließend bei 15 bis 20°C zu einer Lösung von 89,7 g (0,2 mol) des Hydrazons der Formel 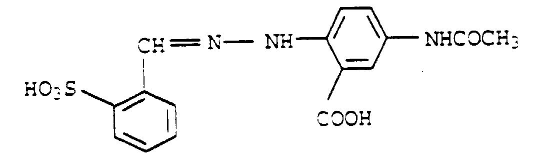 in 200 ml Wasser und 20 ml 50 gew.-%iger wäßriger Natronlauge und 50 g Kupfersulfat getropft. Der pH-Wert wurde während des Zutropfens durch Zugabe von 30 ml 50 gew.-%iger wäßriger Natronlauge bei 6,5 bis 7 gehalten. Die erhaltene Suspension, die das Aminoformazan der Formel 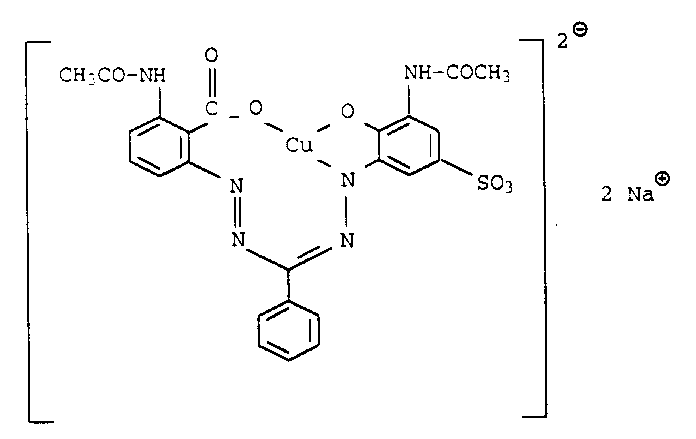 enthielt, wurde im folgenden ohne weitere Reinigung weiterverwendet.
b) Zu der unter Beispiel 5a) hergestellten Suspension wurden 66 ml (1,2 mol) 50 gew.-%ige wäßrige Natronlauge gegeben. Die resultierende Mischung wurde in einem Autoklaven eine Stunde auf 160°C erhitzt (Druck: ca. 4 bar). Nach Beendigung der Reaktion wurde auf 70°C abgekühlt, entspannt und die Reaktionslösung die die freie Aminoverbindung der Formel 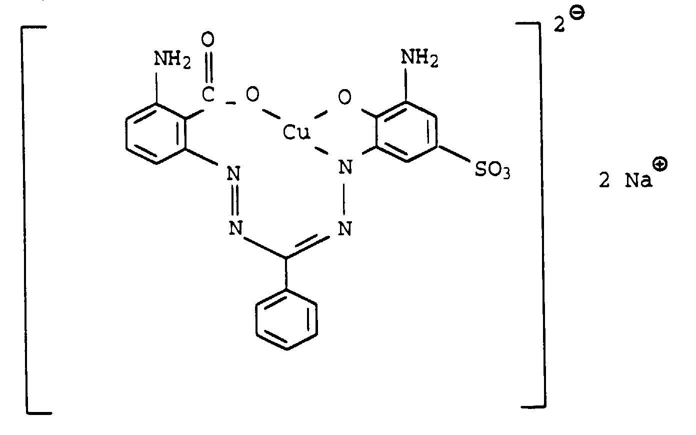 enthielt, klärfiltriert. Man erhielt 2040,5 g Farbstofflösung.
c) Die unter Beispiel 5b) erhaltene Lösung wurde mit Salzsäure auf einen pH-Wert von 5 gestellt. Hierzu wurde bei 5°C eine Lösung von 73,8 g (0,4 mol) Cyanurchlorid in 90 ml Aceton gegeben. Anschließend wurde auf 20°C erwärmt und durch Aussalzen mit 100 g Kaliumchlorid und 300 g Natriumchlorid das Produkt ausgefällt. Nach Absaugen und Trocknen erhielt man 310,5 g der Verbindung der Formel 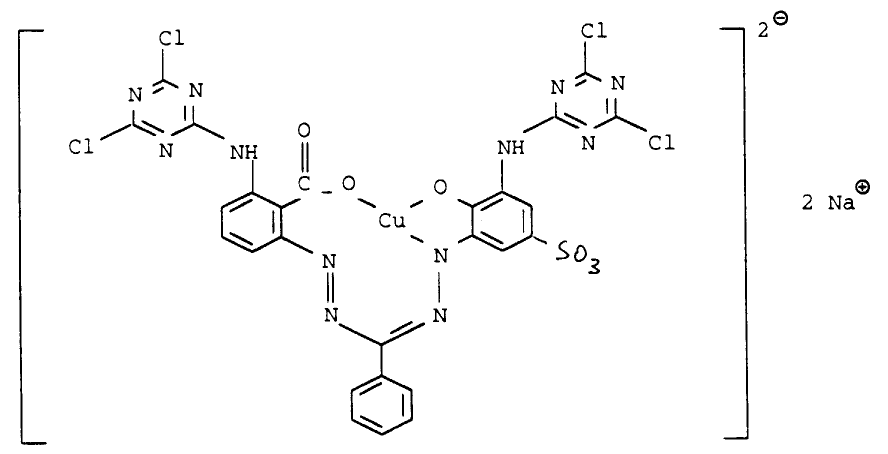 mit einem Gehalt von 47,5 %. Die Ausbeute betrug 76 % d. Th.

In analoger Weise können die im folgenden aufgeführten Aminoformazane erhalten werden.

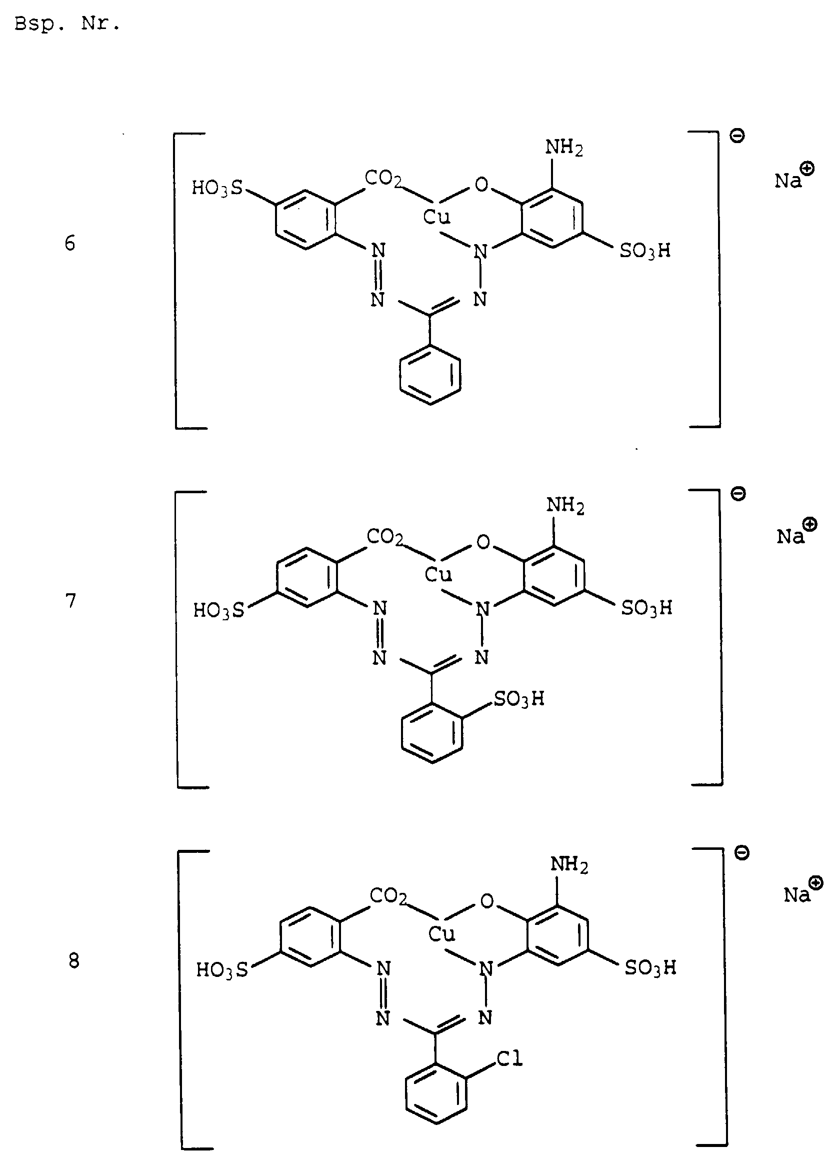

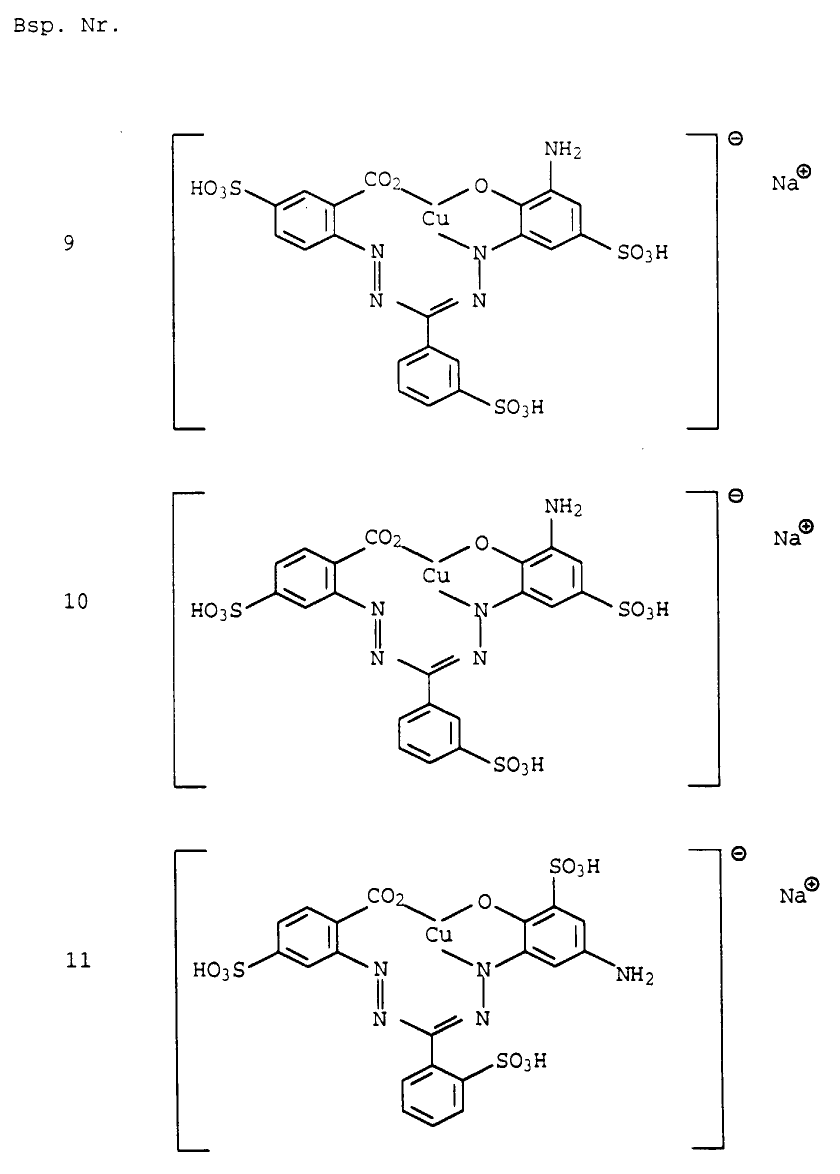

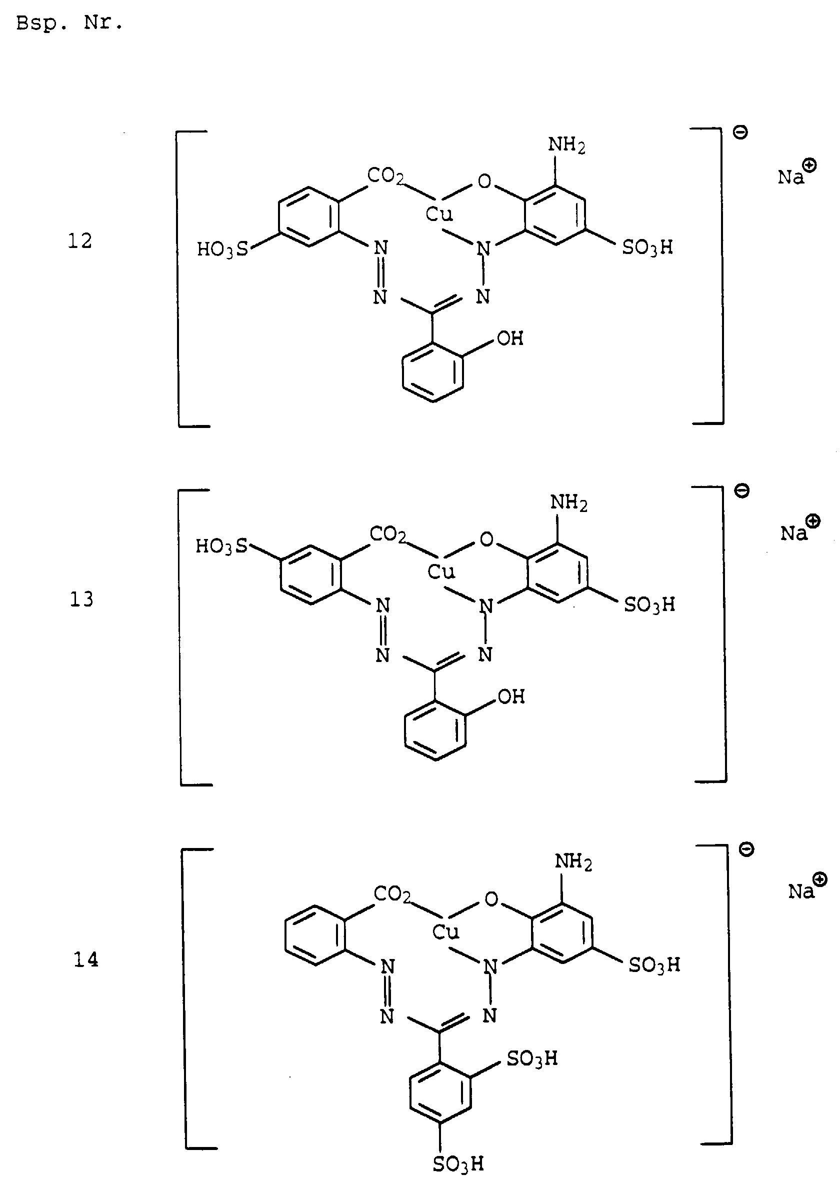

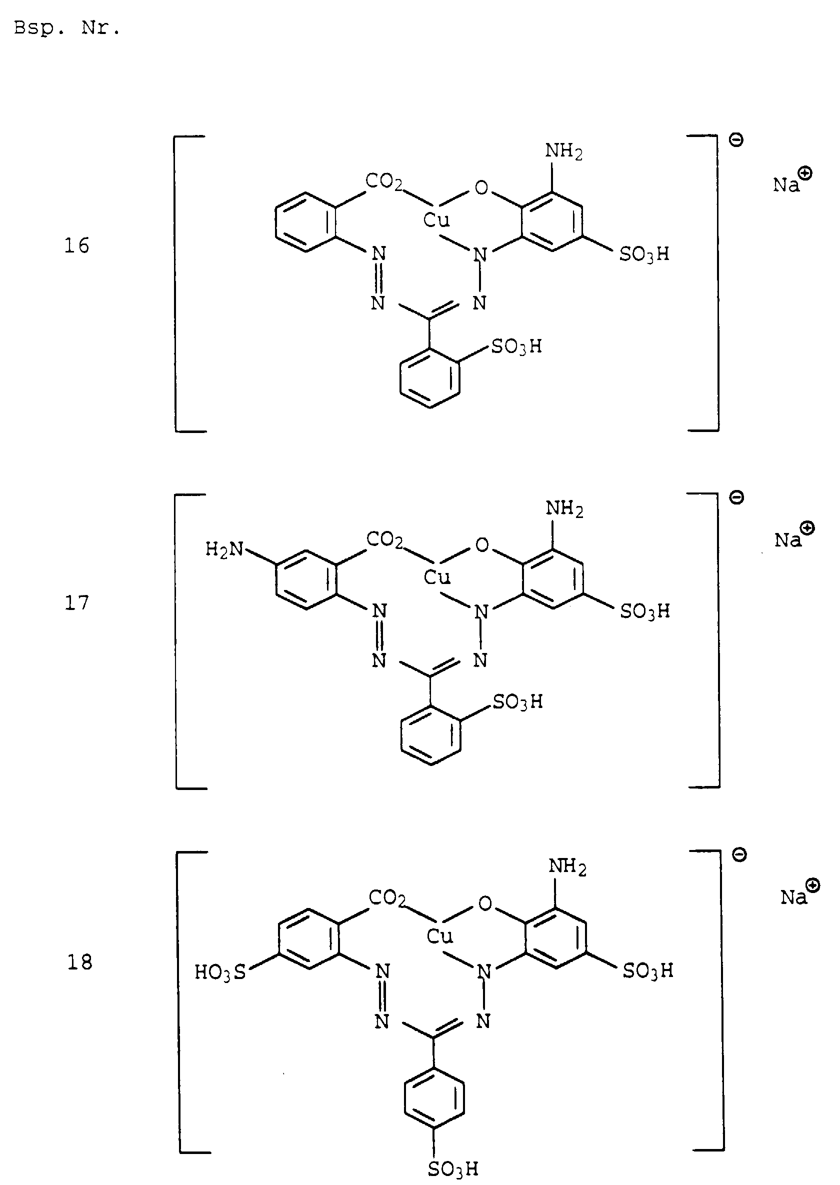

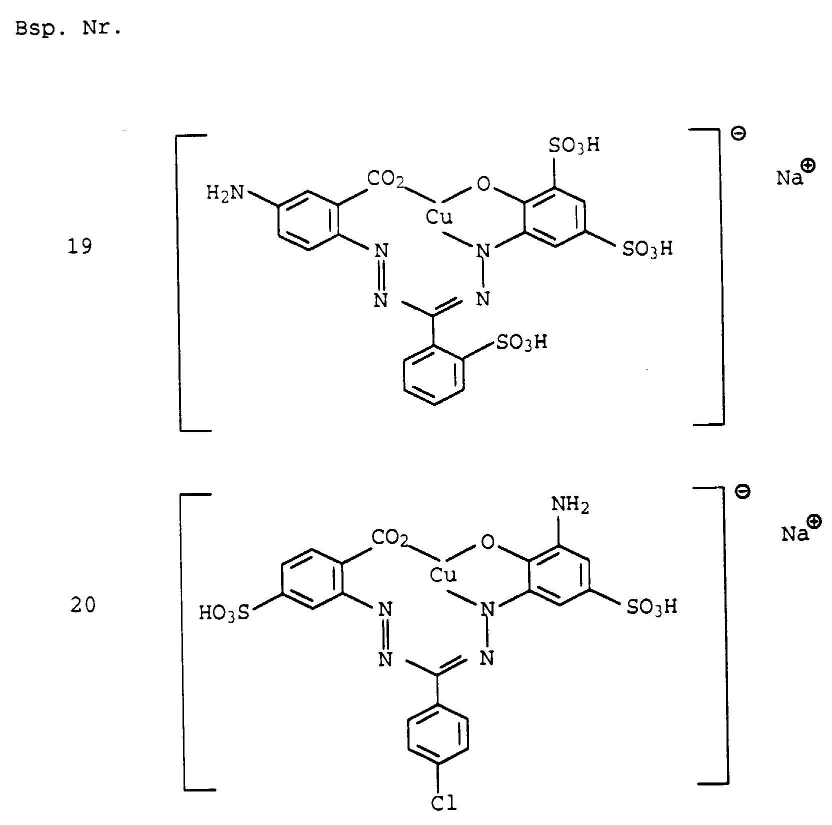

Die Herstellung der Reaktivfarbstoffe aus den obengenannten Aminoformazanen erfolgt durch Umsetzung mit Cyanurchlorid, wie in den voranstehenden Beispielen beschrieben.

## Patentansprüche

1. Verfahren zur Herstellung von Aminoformazanen der Formel I 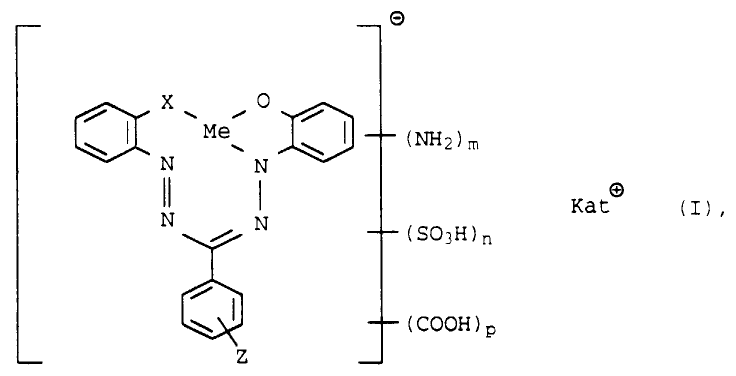 in der
X Sauerstoff oder einen Rest der Formel CO-O oder SO₂-O,
Me Kupfer oder Nickel,
Z Wasserstoff, Halogen oder Hydroxy,
Kat^{⊕} das Äquivalent eines Kations,
m 1 oder 2,
n 1, 2, 3 oder 4 und
p 0, 1 oder 2 bedeuten, mit der Maßgabe, daß die Summe von n+p 1 bis 4 beträgt,
durch alkalische Hydrolyse von acylierten Aminoformazanen der Formel II 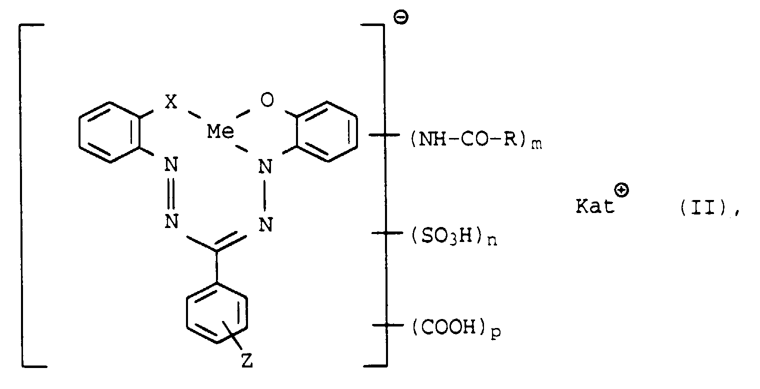 in der R Wasserstoff oder C₁-C₄-Alkyl bedeutet und X, Me, Z, Kat^{⊕} , m, n und p jeweils die obengenannte Bedeutung besitzen, in wäßrigem Medium, dadurch gekennzeichnet, daß man die Hydrolyse bei einer Temperatur von 120 bis 170°C und einem Druck von 3 bis 5 bar in Gegenwart von 1,5 bis 3 mol eines Alkalihydroxids, bezogen auf 1 mol acyliertes Aminoformazan der Formel II, durchführt.

2. Verfahren nach Anspruch 1, dadurch gekennzeichnet, daß man die Hydrolyse bei einer Temperatur von 140 bis 160°C durchführt.

3. Verfahren nach Anspruch 1, dadurch gekennzeichnet, daß man als Alkalihydroxid Natrium- oder Kaliumhydroxid verwendet.

4. Verfahren nach Anspruch 1, dadurch gekennzeichnet, daß X den Rest CO-O und Me Kupfer bedeuten.

5. Verfahren nach Anspruch 1, dadurch gekennzeichnet, daß R Methyl bedeutet.

## Claims

1. A process for preparing aminoformazans of the formula I 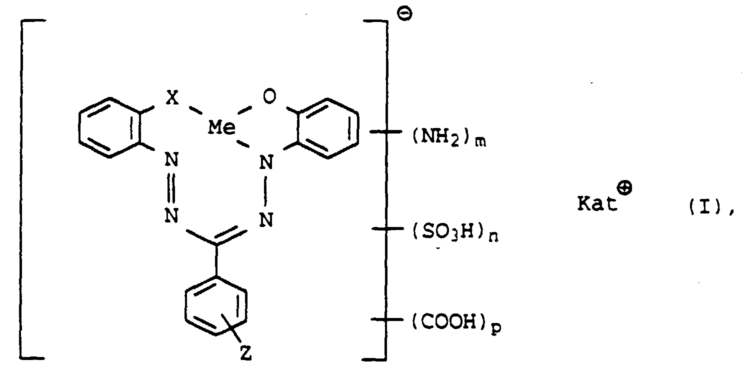 where
X is oxygen or a radical of the formula CO-O or SO₂-O,
Me is copper or nickel,
Z is hydrogen, halogen or hydroxyl,
Cat^{⊕} is the equivalent of a cation,
m is 1 or 2,
n is 1, 2, 3 or 4 and
p is 0, 1 or 2, with the proviso that the sum of n+p is 1 to 4,
by alkaline hydrolysis of acylated aminoformazans of the formula II 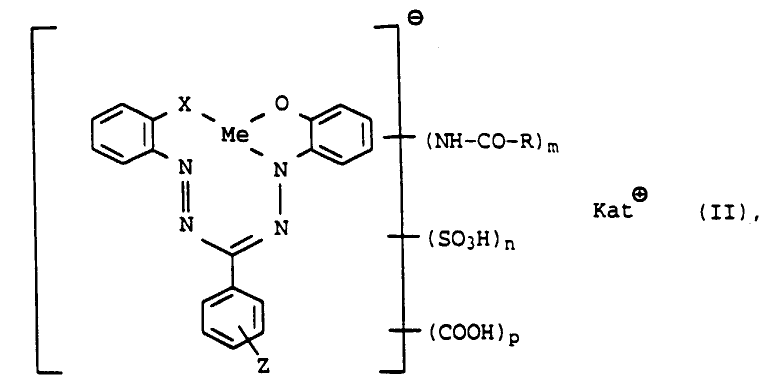 where R is hydrogen or C₁-C₄-alkyl and X, Me, Z, Cat^{⊕} , m, n and p in each case have the abovementioned meanings, in aqueous medium, which comprises carrying out the hydrolysis at from 120 to 170°C and from 3 to 5 bar in the presence of from 1.5 to 3 mol of an alkali metal hydroxide, based on 1 mol of acylated aminoformazan of the formula II.

2. A process as claimed in claim 1, wherein the hydrolysis is carried out at from 140 to 160°C.

3. A process as claimed in claim 1, wherein sodium or potassium hydroxide is used as the alkali metal hydroxide.

4. A process as claimed in claim 1, wherein X is the radical CO-O and Me is copper.

5. A process as claimed in claim 1, wherein R is methyl.

## Revendications

1. Procédé de préparation d'aminoformazans de formule I 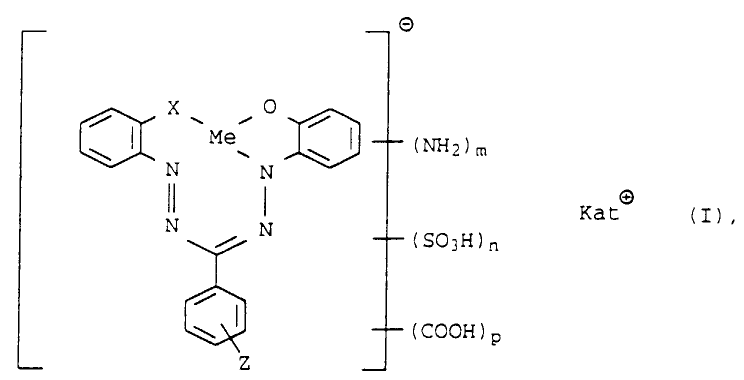 dans laquelle
X représente un atome d'oxygène ou un reste de formule CO-O OU SO₂-O,
Me représente un atome de cuivre ou de nickel,
Z représente un atome d'hydrogène, d'halogène ou un reste hydroxy
Kat^{⊕} est l'équivalent d'un cation,
m est mis pour 1 ou 2,
n est mis pour 1, 2, 3 ou 4 et
p est mis pour 0, 1 ou 2, étant spécifié que la somme de n+p est comprise entre 1 et 4,
par hydrolyse alcaline d'aminoformazans acylés de formule II 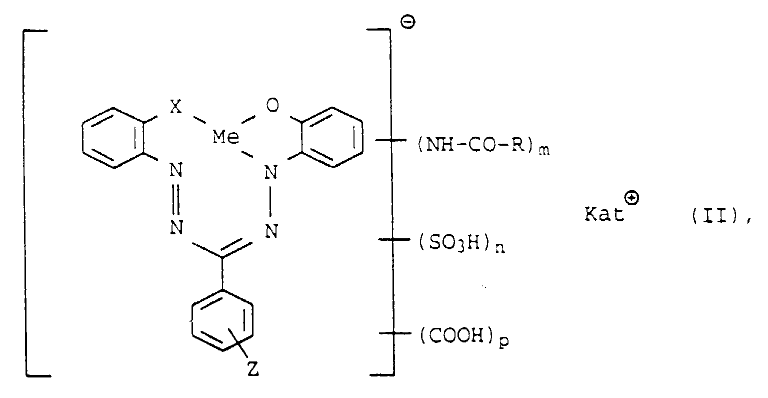 dans laquelle R représente un atome d'hydrogène ou un reste alkyle en C₁-C₄ et X, Me, Z, Kat^{⊕}, m, n et p ont chacun la signification donnée ci-dessus, en milieu aqueux, caractérisé en ce que l'on conduit l'hydrolyse à une température de 120 à 170°C et sous une pression de 3 à 5 bar, en présence de 1,5 à 3 moles d'un hydroxyde de métal alcalin, par rapport à 1 mole d'aminoformazan acylé de formule II,

2. Procédé selon la revendication 1, caractérisé en ce que l'on conduit l'hydrolyse à une température de 140 à 160°C.

3. Procédé selon la revendication 1, caractérisé en ce que l'on utilise, comme hydroxyde de métal alcalin, de l'hydroxyde de sodium ou de potassium.

4. Procédé selon la revendication 1, caractérisé en ce que X représente le reste CO-O et Me représente un atome de cuivre.

5. Procédé selon la revendication 1, caractérisé en ce que R représente un reste méthyle.
